# EUROPEAN PATENT SPECIFICATION

(11) **EP 4 413 976 B1**
(45) Date of publication and mention of the grant of the patent: **13.08.2025**
(21) Application number: 23155431.2
(22) Date of filing: 07.02.2023
(51) Int. Cl.: A61K 9/14, A61K 9/16, A61K 31/5685, A61K 31/573, A61K 9/20

(54) **A NEW VAMOROLONE PREPARATION EXHIBITING IMPROVED SOLUBILITY**
VAMOROLON-ZUBEREITUNG MIT VERBESSERTER LÖSLICHKEIT
NOUVELLE PREPARATION DE VAMOROLONE PRESENTANT UNE SOLUBILITE AMELIOREE

(43) Date of publication of application: 14.08.2024
(73) Proprietor: Santhera Pharmaceuticals (Schweiz) AG, 4133 Pratteln (CH)
(72) Inventor: ALLIERI, Brigida, 37138 Verona (IT); CHALLIS, Bradley, Nottingham, NG10 2BJ (GB); CHOPRA, Reenu, Nottingham, NG12 4HX (GB); DIAZ, Liliana, 4125 Riehen (CH); HAUSMANN, Rudolf, 4052 Basel (CH)
(74) Representative: Grünecker Patent- und Rechtsanwälte PartG mbB

(56) References cited:
- WO-A2-2009/155056
- US-A1- 2020 281 942
- US-A1- 2023 002 439

## Description

Glucocorticoids are standard of care for many inflammatory conditions, but long-term use is associated with a broad array of side effects. Recently, it was found that Δ9,11 steroid analogs hold promise as a source of safer agents for treating chronic inflammatory disorders.

Vamorolone is one of these Δ9,11 steroid analogs. Vamorolone is a synthetic glucocorticoid corticosteroid, also known as VB-15, VBP-15, 16α-methyl-9,11-dehydroprednisolone, or 17α,21-dihydroxy-16α-methylpregna-1,4,9(11)-triene-3,20-dione:

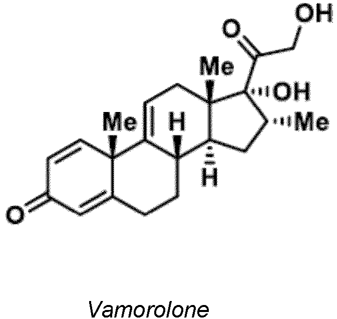

Pre-clinical data have shown that Vamorolone binds potently to the glucocorticoid receptor and has anti-inflammatory effects similar to traditional glucocorticoid drugs. Testing of multiple mouse models of inflammatory states has shown efficacy similar to prednisolone, and dramatically improves side effect profiles, including loss of growth stunting and loss of bone fragility. Recent clinical data have confirmed these beneficial effects.

US 2020/0281942 discloses an aqueous oral pharmaceutical suspension composition comprising crystalline Vamorolone. Vamorolone has limited solubility in aqueous media. Solubility is rather independent from pH due to a lack of ionizable groups in the physiological pH range. The thermodynamic solubility at 37°C in aqueous buffers is between 33 and 37 µg/ml over a range of pH 1 to pH 7.

The only available pharmaceutically dosage form of Vamorolone to date is an aqueous oral pharmaceutical suspension including 300 mg Vamorolone per 7.5 ml for pediatric use. Typically, pediatric patients receive a bottle containing 100 ml of this aqueous oral suspension. Depending on the patient's body weight, one unit of the aqueous oral suspension product might not be enough to cover one month of treatment. For these patients, there is a need to develop a more convenient immediate release oral solid dosage form of suitable size, offering good physical and chemical stability upon storage, and facilitating simple and accurate dosing.

Poor aqueous solubility and slow dissolution rate are known to lead to low bioavailability or negatively affect pharmacokinetic performance and limit the orally absorbed dose. Therefore, there is a need for oral formulations containing Vamorolone with increased dissolution rate and solubility, capable of providing suitable bioavailability at a palatable oral solid dose(s).

Overall, there is a need for overcoming the problems arising from the Vamorolone formulation provided by US 2020/0281942. Especially, there is a need for providing higher doses of Vamorolone in a convenient pharmaceutical dosage form, preferably an immediate release solid dosage form, containing Vamorolone with enhanced solubility and dissolution rate when compared to crystalline drug, which is able to deliver Vamorolone as a stable formulation, more preferably with improved bioavailability at an optimized dose. Further, there is a need to provide an immediate release solid oral dosage form containing Vamorolone.

### SUMMARY OF THE INVENTION

The present invention as defined in the appended claims fulfils this need by providing Vamorolone as an amorphous solid composition. The composition of the present invention comprises an amorphous solid dispersion of Vamorolone in a polymer matrix, i.e. amorphous Vamorolone dispersed in a polymer matrix.

The terms "composition of the present invention" and the "amorphous solid dispersion of the present invention" are used as synonyms through the specification.

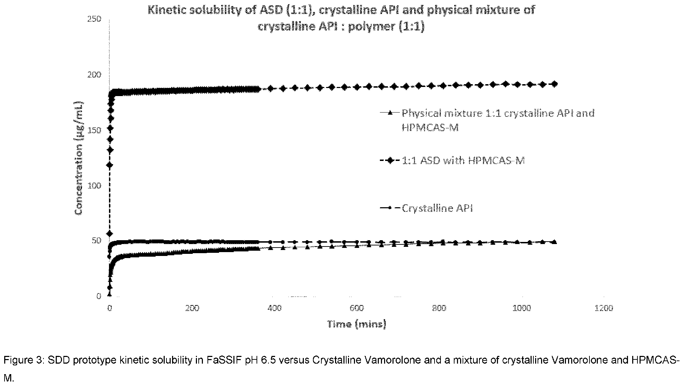

The presence of the polymer alone in a composition comprising Vamorolone, but not as amorphous solid dispersion of the present invention does not result in an increase of solubility as is seen for the solid dispersion. Just blending Vamorolone (be it crystalline or amorphous) with a polymer does lead to the same solubility as amorphous Vamorolone alone - see Figure 3.

The amorphous solid dispersion of the present invention, hence, was found to be a formulation with increased dissolution properties and that is capable of stabilizing the solubility of Vamorolone in aqueous media.

It has been surprisingly found that the composition of the present invention exhibits a drastically enhanced kinetic solubility in aqueous media, which can allow for a better bioavailability, regardless of the co-administration of food, compared to the Vamorolone compositions in the prior art.

It has further been surprisingly found that the composition of the present invention comprising an amorphous solid dispersion of Vamorolone dissolved in an aqueous medium is stable over a long time period, without recrystallization, in a form of a supersaturated aqueous solution, so that the solubilized form can be efficiently absorbed *in vivo.*

The composition of the present invention comprising an amorphous solid dispersion of Vamorolone is preferably obtained by spray drying. This technology was found to be superior compared to other alternatives such as hot melt extrusion, since it allows for a high drug load while preventing the composition from degradation and demonstrates good processability to pharmaceutical dosage forms.

A suitable polymer or a mixture of suitable polymers are employed to stabilize Vamorolone in the amorphous state. In general, the polymer is pharmaceutically acceptable. Typical examples of polymers include cellulose or cellulose derivatives, povidone and copovidone.

Polymers are preferably selected from hydroxypropylmethylcellulose (HPMC), copovidone (polyvinylpyrrolidone-vinyl acetate copolymer), and hydroxypropylmethylcellulose acetate succinate (HPMCAS, grade L, M or H). In most preferred embodiments, the Vamorolone polymer is hydroxypropylmethylcellulose acetate succinate.

In specific embodiments, the polymer is selected from hydroxypropylmethyl cellulose acetate succinate grade L, H or M. In one preferred embodiment, the polymer is hydroxypropylmethyl cellulose acetate succinate (HPMCAS) grade M that dissolves at pH values above 6.0. Preferably, HPMCAS grade L, H or M is characterized by an acetyl content of about 7 - 11% and a succinoyl content of about 10-14%.

The kinetic solubility of Vamorolone in the amorphous state is increased in the presence of one or more of the polymers of the invention, leading to supersaturated solutions when dissolving the composition of the present invention. Such supersaturated solutions of Vamorolone are stable for at least 10 hours in a concentration of more than 50 µg/ml, preferably more than 100 µg/ml, even more preferably more than 150 µg/ml, even more preferably more than 200 µg/ml, even more preferably more than 250 µg/ml, even more preferably more than 300 µg/ml and most preferably more than 350 µg/ml. All concentrations above refer to solubility in an aqueous medium at 37°C. Typically the aqueous medium is fasted stated simulated intestinal fluid (FASSIF) at 37°C.

In one embodiment, the amorphous solid dispersion of the present invention comprises at least 20 wt% of Vamorolone by weight of the amorphous solid dispersion and 80 wt% of total polymer by weight of the amorphous solid dispersion. Some amorphous solid dispersions comprise from about 20 wt% to about 80 wt% of Vamorolone by weight of the amorphous solid dispersion. Preferably, the amorphous solid dispersions of the present invention contain about 30 wt% to 70 wt% of Vamorolone. More preferably, the amorphous solid dispersions contain about 40 wt% to 60 wt% of Vamorolone. More preferably, the amorphous solid dispersions contain about 45 wt% to 55 wt% of Vamorolone. The total amount of polymers preferably is 100 wt% minus the Vamorolone amount, e.g. total polymer amount of 80 wt% to 20 wt% of the amorphous solid dispersion, if the Vamorolone amount is 20 wt% to 80wt% of the amorphous solid dispersion. The same calculation applies to all the preferred Vamorolone amounts as set forth above. In another preferred embodiment, the invention relates to an amorphous solid dispersion containing an amount of 50 wt% of Vamorolone, in a 1:1 ratio with the total amount of polymer.

The polymers in an alternatively preferred embodiment have an amount of 99 wt% minus the Vamorolone amount, e.g total polymer.in an amount of 79 wt% to 19 wt% of the amorphous solid dispersion, if the Vamorolone amount is 20 wt% to 80wt% of the amorphous solid dispersion. The total polymer amount is 69 wt% to 29 wt% of the amorphous solid dispersion, if the Vamorolone amount is 30 wt% to 70 wt% of the amorphous solid dispersion. The total polymer amount is 59 wt% to 39 wt% of the amorphous solid dispersion, if the Vamorolone amount is 40 wt% to 60 wt% of the amorphous solid dispersion. The total polymer amount is 54 wt% to 34 wt% of the amorphous solid dispersion, if the Vamorolone amount is 45 wt% to 55 wt% of the amorphous solid dispersion.

The polymers in still another preferred embodiment have an amount of 97 wt% minus the Vamorolone amount, e.g. total polymer in an amount of 77 wt% to 17 wt% of the amorphous solid dispersion, if the Vamorolone amount is 20 wt% to 80wt% of the amorphous solid dispersion. The total polymer amount is 67 wt% to 27 wt% of the amorphous solid dispersion, if the Vamorolone amount is 30 wt% to 70 wt% of the amorphous solid dispersion. The total polymer amount is 57 wt% to 37 wt% of the amorphous solid dispersion, if the Vamorolone amount is 40 wt% to 60 wt% of the amorphous solid dispersion The total polymer amount is 52 wt% to 32 wt% of the amorphous solid dispersion, if the Vamorolone amount is 45 wt% to 55 wt% of the amorphous solid dispersion.

In yet another preferred embodiment, the invention relates to an amorphous solid dispersion containing amorphous Vamorolone in a polymer matrix together with one or more further active ingredients.

Higher amounts of Vamorolone, above 67 wt% of the solid dispersion, have been linked to physical instability. When exposed to stress conditions during storage (40°C/75% relative humidity, for 1 week) partial re-crystallization of the amorphous form was observed, then. Also, despite an initial increase in kinetic solubility in water or aqueous media, the ability to maintain supersaturation was negatively impacted when Vamorolone loads of about 67 wt% or higher in the solid dispersion were used.

In preferred embodiments, the composition of the invention does not comprise any additional ingredients apart from polymer and Vamorolone. In another embodiment, the composition of the invention comprises up to 3 wt%, or up to 2 wt% or up to 1 wt% of total amount of any additional ingredients. Additional ingredients are for example additional ingredients for stabilizing Vamorolone in the amorphous phase.

In still further embodiments, a pharmaceutical composition is provided comprising the amorphous solid dispersion of Vamorolone and one or more pharmaceutically acceptable excipients. Such a pharmaceutical composition might contain pharmaceutically acceptable excipients of different functional categories, including, but not limited to, diluents, binders, disintegrants, lubricants, glidants, surfactants and polymeric film coatings

In one particular embodiment the pharmaceutical composition is a solid oral dosage form. In a more preferred embodiment, the solid dosage form is an immediate release tablet or capsule for oral administration. Such a tablet or capsule might contain pharmaceutically acceptable excipients of different functional categories, including, but not limited to, diluents, binders, disintegrants, lubricants, glidants, surfactants and polymeric film coatings.

In a further preferred embodiment, the amorphous Vamorolone dispersion of the present invention is prepared by spray drying. Accordingly, the present invention provides a method for preparing a Vamorolone composition of the present invention, i.e. Vamorolone as a solid dispersion of amorphous Vamorolone in a polymer matrix, by spray drying. Spray drying is well known in the art. The method e.g. comprises atomizing a solution containing the drug substance Vamorolone and a polymer, optionally together with suitable excipients to generate fine droplets that are introduced into a chamber, where they are contacted with a hot drying gas and dried through solvent evaporation to form particles. The particles may subsequently be separated from the drying medium, e.g. using a cyclone or a bag filter. Hence, the spray drying process e.g. comprises: atomizing the feed liquid comprising Vamorolone and polymer or other suitable excipients, contacting the so obtained droplets with a hot drying medium, drying of the droplets into particles and separating the particles from the drying medium. The powder obtained may preferably undergo a secondary drying step to ensure the removal of residual solvents to acceptable levels.

It has been surprisingly found that other methods were largely unsuccessful in preparing the composition of the present invention. Experiments using hot melt extrusion failed to obtain stable amorphous solid dispersions of Vamorolone with suitable drug loads. E.g. drug loads of about 20 wt% Vamorlone in the dispersion or lower were necessary to obtain and stabilize the amorphous form of Vamorolone. Such drug loads are not compatible with a suitable tablet size that is convenient for the patient in terms of swallowability. Conditions required for higher drug loads, on the other hand, led to significant degradation of the drug substance.

One embodiment of preparing the solid dispersion of the invention using spray drying comprises dissolving the drug and a polymer in an organic solvent or a mixture of organic solvents, and spraying the obtained solution into a stream of heated gas to remove the organic solvent.

With spray drying, it is possible to co-precipitate a drug substance with a polymer into a stable amorphous solid dispersion thanks to the fast drying rate that enables the drug substance to be captured in amorphous form.

In another aspect of the invention, a pharmaceutical composition comprising the Vamorolone of the present invention is provided. More preferably, the present invention provides a solid dosage form, preferably a tablet or coated tablet. In another embodiment, the solid dosage form is a capsule. The capsule may be filled with a powder, granules, pellets, multiparticulates or mini-tablets comprising the composition of the present invention.

Further provided is a solid oral pharmaceutical composition comprising a Vamorolone composition of the present invention, as an amorphous solid dispersion; and at least one filler, one disintegrant, binder glidant, wetting agent and/or one lubricant. In preferred embodiments, a film coating may be added. Also provided are methods for preparing such solid oral pharmaceutical composition.

In another aspect, the present invention provides Vamorolone compositions of the present invention for use in methods of treating of a disease selected from
a) asthma or chronic obstructive pulmonary disease,
b) a skin disease,
c) Sjogren's syndrome ,
d) arthritis, or
e) muscular wasting.

Preferably, the muscular wasting disease is muscular dystrophy In some embodiments, the muscular dystrophy is chosen from Duchenne muscular dystrophy, Becker muscular dystrophy, limb girdle muscular dystrophy, congenital muscular dystrophy, facioscapulohumeral muscular dystrophy, myotonic muscular dystrophy, oculopharyngeal muscular dystrophy, distal muscular dystrophy, and Emery Dreifuss muscular dystrophy.

Most preferred, the muscular dystrophy is Duchenne muscular dystrophy.

Further provided is the use of a polymer to improve the solubility of Vamorolone in an aqueous medium by dispersing Vamorolone in a solid amorphous form in the polymer. In this aspect of the invention, at least 50 wt% of the total Vamorolone amount shall be dispersed amorphous Vamorolone; more preferably at least 60 wt% of the total Vamorolone amount shall be dispersed amorphous Vamorolone; even more preferably at least 70 wt% of the total Vamorolone amount shall be dispersed amorphous Vamorolone; even more preferably at least 80 wt% of the total Vamorolone amount shall be dispersed amorphous Vamorolone; even more preferably at least 90 wt% of the total Vamorolone amount shall be dispersed amorphous Vamorolone, even more preferably at least 95 wt% of the total Vamorolone amount shall be dispersed amorphous Vamorolone; even more preferably at least 97 wt% of the total Vamorolone amount shall be dispersed amorphous Vamorolone; and most preferably at least 99 wt% of the total Vamorolone amount shall be dispersed amorphous Vamorolone. All amounts are amounts of amorphous Vamorolone per total Vamorolone. The Vamorolone dispersion is preferably obtained by spray drying. The Vamorolone is preferably the Vamorolone in a composition of the present invention as defined in any of the embodiments set forth above.

### BRIEF DESCRIPTION OF THE FIGURES

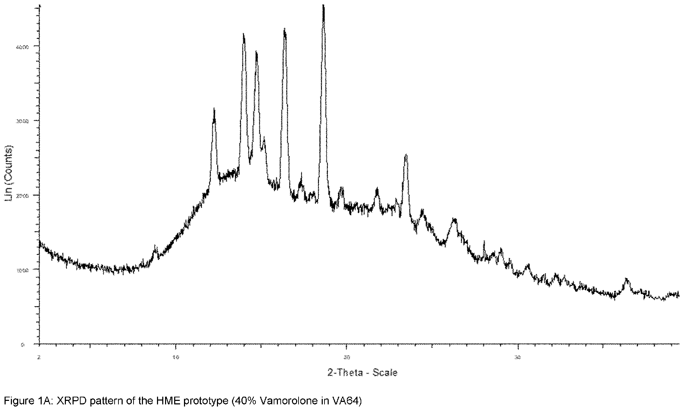

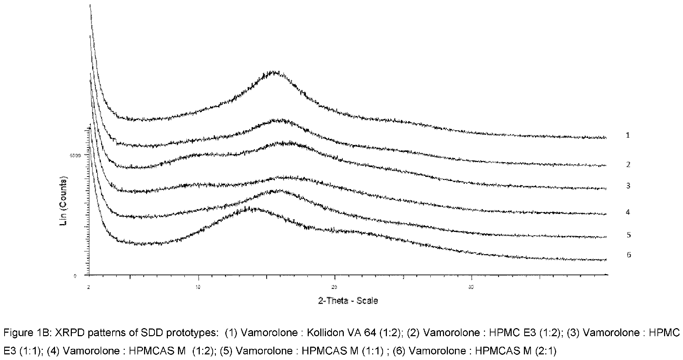

Figure 1A and 1B depict X-ray diffractograms obtained for solid dispersion prototypes obtained by hot melt extrusion (1A) and spray drying (1B). For hot melt extrusion, the choice of the polymer (copovidone VA64) was based on the outcome of preformulation studies (miscibility experiments) employing differential scanning calorimetry (DSC) that evaluated the following polymers: copovidone VA64, mixtures of copovidone VA64/poloxamer, mixtures of copovidone VA64/HPMC E3, HPMCAS-L, and HPMCAS-M. The results of hot melt extrusion with the polymer that exhibited the best miscibility were unsatisfactory for a drug load of 40% or more, since signs of crystallinity were evident in the diffractograms. In addition, HPLC analysis of the samples revealed degradation of the drug substance.

For spray drying, however, XR patterns typical of amorphous materials were obtained for several polymers: HPMCAS-M (67%, 50% and 33% drug load), HPMC E3 (drug load 50% and 33%), and copovidone VA64 (drug load 33%), thus suggesting that the conditions applied during spray drying, compared to hot melt extrusion, are particularly advantageous to obtain stable amorphous solid dispersions of Vamorolone.

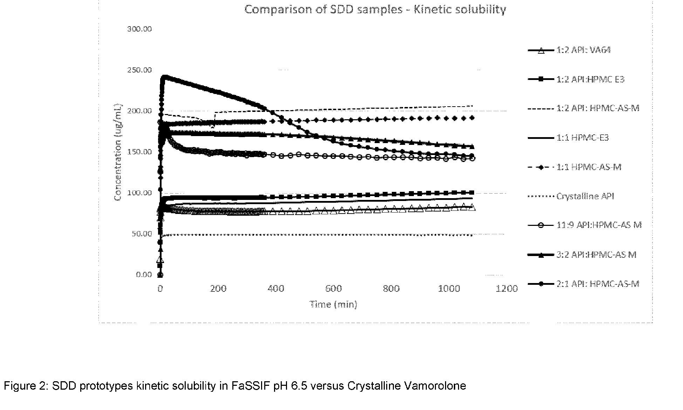

Figure 2 shows on overlay of kinetic solubility profiles obtained for eight spray dried amorphous solid dispersion prototypes. In fasted state simulated intestinal fluid (FaSSIF). The solubility profile of micronized crystalline Vamorolone has also been included for comparison purposes. All prototypes exhibited an increased solubility compared to crystalline Vamorolone, however, the performance of the different polymers was unexpectedly diverse, being ranked as: HPMC-AS M > HPMC-E3 > Kollidon VA64. In addition, the ability of Vamorolone to remain solubilized by the polymer in a supersaturated solution is affected by the different drug loadings. For the polymer with the best performance, HPMC-AS M, at higher polymer loadings (and hence lower API loadings), the solubilized state was maintained and no precipitation of Vamorolone was observed. At higher drug loads of around 67% or more, Vamorolone is at a much higher risk of precipitation as the drug to polymer ratio is reduced and less polymer is available to stabilize the drug.

Figure 3 shows comparative kinetic solubility profiles obtained for crystalline Vamorolone, spray dried amorphous solid dispersion prototype (Vamorolone: HPMCAS M 1:1) and a physical mixture of Vamorolone and HPMCAS M 1:1. The presence of the polymer alone does not result in an increase of solubility as seen for the solid dispersion.

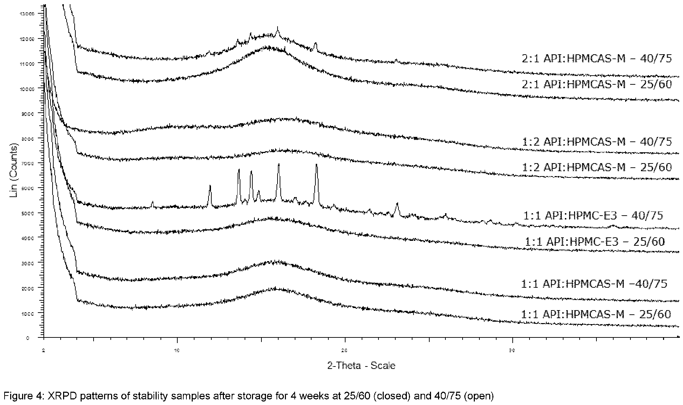

Figure 4 shows XRPD stability data for spray dried prototypes signs of crystallization are evident after 4 weeks at 40/75 for the prototype obtained with HPMC E3 and for the prototype containing the highest drug load (API: HPMCAS-M 2:1).

### DETAILED DESCRIPTION

VAMOROLONE: As used herein, "Vamorolone" refers to 17α,21-dihydroxy-16α-methylpregna-1,4,9(11)-triene-3,20-dione (also known as VBP15) and has the structure:

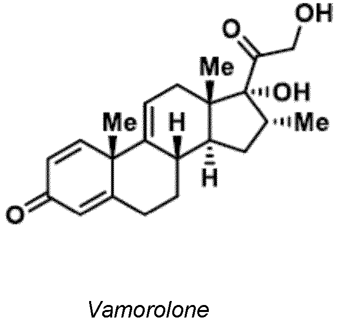

CRYSTALLINE VAMOROLONE: Crystalline Vamorolone refers to the drug substance that includes Vamorolone crystals in an amount that can be detected by electron microscopy or by XRPD. XRPD (X-Ray Powder Diffraction) is an experimental technique that uses the diffraction of X-rays on powder or microcrystalline samples for the structural characterization of materials analysis.

AMORPHOUS VAMOROLONE: Amorphous Vamorolone refers to Vamorolone drug substance that exists predominantly in the amorphous state, i.e. does not contain crystalline Vamorolone in an amount that can be detected by XRPD. Preferably, the amount of crystalline Vamorolone in amorphous Vamorolone is less than 10 wt%, more preferably less than 5 wt%, more preferably less than 3wt%, more preferably less than 2 wt% or most preferably less than 1 wt%. Ideally, amorphous Vamorolone is free of any crystalline form.

ADMINISTERING: As used herein, "administering" means to provide a compound or other therapy, remedy, or treatment such that an individual internalizes a compound.

DISEASE: As used herein, the term "disease" is intended to be generally synonymous, and is used interchangeably with, the terms "disorder" and "condition" (as in medical condition), in that all reflect an abnormal condition of the human or animal body or of one of its parts that impairs normal functioning, is typically manifested by distinguishing signs and symptoms, and causes the human or animal to have a reduced duration or quality of life.

NF-κB-MEDIATED DISEASE: Vamorolone can be used to treat any type of NF-κB-MEDIATED DISEASE. As used herein, the term "NF-κB-mediated disease" refers to a disease having a significant and pathologic inflammatory component that can be addressed by inhibition of NF-κB. The term "NF-κB-mediated disease" also refers to the following diseases, even though the compounds disclosed herein exert their effects through biological pathways and/or processes other than NF-κB: muscular dystrophy, arthritis, traumatic brain injury, spinal cord injury, sepsis, rheumatic disease, cancer atherosclerosis, type 1 diabetes, type 2 diabetes, leptospirosis renal disease, glaucoma, retinal disease, ageing, headache, pain, complex regional pain syndrome, cardiac hypertrophy, muscle wasting, catabolic disorders, obesity, fetal growth retardation, hypercholesterolemia, heart disease, chronic heart failure, ischemia/reperfusion, stroke, cerebral aneurysm, angina pectoris, pulmonary disease, cystic fibrosis, acid-induced lung injury, pulmonary hypertension, asthma, chronic obstructive pulmonary disease, Sjogren's syndrome, hyaline membrane disease, kidney disease, glomerular disease, alcoholic liver disease, gut diseases, peritoneal endometriosis, skin diseases, nasal sinusitis, mesothelioma, anhidrotic ectodermal dysplasia-ID, Behcet's disease, incontinentia pigmenti, tuberculosis, asthma, Crohn's disease, colitis, ocular allergy, appendicitis, Paget's disease, pancreatitis, periodontitis, endometriosis, inflammatory bowel disease, inflammatory lung disease, silica-induced diseases, sleep apnea, AIDS, HIV-1, autoimmune diseases, antiphospholipid syndrome, lupus, lupus nephritis, familial Mediterranean fever, hereditary periodic fever syndrome, psychosocial stress diseases, neuropathological diseases, familial amyloidotic polyneuropathy, inflammatory neuropathy, Parkinson's disease, multiple sclerosis, Alzheimer's disease, amyotrophic lateral sclerosis, Huntington's disease, cataracts, and hearing loss.

PHARMACEUTICAL COMPOSITION: As used here, "pharmaceutical composition" means a composition comprising at least one active ingredient, such as Vamorolone, whereby the composition is amenable to investigation for a specified, efficacious outcome in a mammal (for example, without limitation, a human).

PURE: As used herein, the term "pure" means about 90-100%, preferably 95-100%, more preferably 98-100% (wt/wt) or 99-100% (wt/wt) pure compound; e.g. less than about 10%, less than about 5%, less than about 2% or less than about 1% impurity is present. Such impurities include, e.g., degradation products, oxidized products, epimers, solvents, and/or other undesirable impurities. In practice, the drug substance specification includes 98.0-102.0 wt% as determined in a conventional assay.

THERAPEUTICALLY ACCEPTABLE: As used herein, the term "therapeutically acceptable" refers to those compounds (or salts, prodrugs, tautomers, zwitterionic forms, etc.) suitable for use in contact with the tissues of patients without undue toxicity, irritation, and allergic response, are commensurate with a reasonable benefit/risk ratio, and are effective for their intended use.

THERAPEUTICALLY EFFECTIVE: As used herein, the phrase "therapeutically effective" is intended to qualify the amount of active ingredient used in the treatment of a disease or disorder. It represents an efficacious dose for the patient to reduce or eliminate symptoms of the disease to be treated.

TREATMENT: As used herein, "treating," "treatment," and the like means ameliorating a disease so as to reduce or eliminate its cause, its progression, its severity, or one or more of its symptoms, or otherwise beneficially alter the disease in a subject. Treatment may also be preemptive in nature, i.e., it may include prophylaxis of disease in a subject exposed to or at risk for the disease. Prevention of a disease may involve complete protection from disease, for example as in the case of prevention of infection with a pathogen, or may involve prevention of disease progression, for example from prediabetes to diabetes. For example, prevention of a disease may not mean complete foreclosure of any effect related to the diseases at any level, but instead may mean prevention of the symptoms of a disease to a clinically significant or detectable level. Prevention of diseases may also mean prevention of progression of a disease.

AMORPHOUS SOLID DISPERSION (ASD): is a system in which an active pharmaceutical ingredient (API), e.g. Vamorolone, is embedded largely amorphously, preferably free of any crystalline form, into a solid matrix of one or more polymers. In the present invention, preferably at least 95 wt% of the total Vamorolone amount are dispersed amorphous Vamorolone, and most preferably at least 99 wt% of the total Vamorolone amount are dispersed amorphous Vamorolone. Ideally, 100 wt% of the total Vamorolone amount are dispersed amorphous Vamorolone. All these amounts are amounts of amorphous Vamorolone per total Vamorolone.

SPRAY DRYING (SD) is a technology that involves the transformation of a liquid material (solution or suspension) into the solid form comprising drying of a solution previously atomized into small droplets.

HOT MELT EXTRUSION (HME) involves the mixing of active compound and excipients (mainly thermoplastic polymers) under thermal and mechanical stress followed by extrusion through a die, often with the aim of melting or dispersing the drug in the polymeric excipient to obtain a product in which the active compound is in an amorphous state,

### Vamorolone as solid dispersion in polymer

Provided herein is a composition comprising an amorphous Vamorolone. Vamorolone may be prepared e.g. according to US 2020/0281942.

The composition of the present invention comprises amorphous Vamorolone in an amorphous solid dispersion with a polymer.

In a further preferred embodiment, the amorphous Vamorolone of the present invention is prepared by spray drying.

In a further preferred embodiment, the composition of the present invention is stable for of at least 2 weeks when stored at 40°C/75% relative humidity in an open container.

In a further preferred embodiment, the composition of the present invention comprises at least 20 wt% Vamorolone, preferably at least 30 wt% Vamorolone, more preferably at least 40 wt% Vamorolone, even more preferably at least 50 wt% by weight of the composition. In any of these embodiments the composition of the invention is preferably an amorphous solid dispersion comprising Vamorolone and a polymer or a mixture of polymers. In another preferred embodiment the composition of the present invention further comprises a wetting agent.

In a further preferred embodiment, the composition of the present invention comprises not more than 80 wt% Vamorolone, preferably not more than 70 wt% Vamorolone, more preferably not more than 67 wt% Vamorolone, even more preferably not more than 60 wt % Vamorolone by weight of the composition. In any of these embodiments the composition of the invention is preferably an amorphous solid dispersion comprising Vamorolone and a polymer.

In one aspect of the present invention, the particle size distribution of the Vamorolone composition of the present invention, i.e. Vamorolone in an amorphous solid dispersion with a polymer, is defined as follows: d10: up to 6 µm, d50: up to 15 µm and d90: up to 35 µm.

In another aspect of the invention, the amorphous solid dispersion composition of the present invention has a BET surface area of between 800 and 1.200 m^{2/}g, preferably between 900 and 1.00 m²/g.

### Pharmaceutical Composition comprising Vamorolone

In another aspect of the invention, a pharmaceutical composition comprising the Vamorolone as amorphous solid dispersion of the present invention is provided.

Preferably, the present invention provides an oral pharmaceutical composition comprising Vamorolone.

In some embodiments, the oral pharmaceutical composition comprises Vamorolone as an amorphous solid dispersion (ASD), in an amount of ASD between about 20 w% and about 80% wt% per total amount of the pharmaceutical composition. In some embodiments, the oral pharmaceutical composition comprises an ASD of Vamorolone in an amount of between about 25% and about 75% wt% per total amount of the pharmaceutical composition. In some embodiments, the oral pharmaceutical composition comprises an ASD of Vamorolone in an amount of between about 30 wt% and about 70% wt% per total amount of the pharmaceutical composition. In some embodiments, the oral pharmaceutical composition comprises an ASD containing Vamorolone in an amount of between about 40 wt% and about 60% wt% per total amount of the pharmaceutical composition. Preferably, in the pharmaceutical composition, the amount of amorphous Vamorolone in the ASD is between 40 wt% and 60 wt% per the amount of the ASD per total amount of the pharmaceutical composition. More preferably, in the pharmaceutical composition, the amount of amorphous Vamorolone in the pharmaceutical composition is between 20 wt% and 40% per the total amount of the pharmaceutical composition.

In some embodiments, the ASD is used for the manufacture of a pharmaceutical composition that further comprises at least one pharmaceutically acceptable ingredient, e.g. a filler. In some embodiments, the filler is selected from lactose, calcium phosphates, sorbitol, mannitol, maltitol, lactitol, cellulose, microcrystalline cellulose, starch, pregelatinized starch, sucrose, or any combination thereof. In one embodiment, the pharmaceutical composition comprises at least one pharmaceutically acceptable ingredient, e.g. filler, in an amount of up to 70 wt%, up to 50wt%, up to 35 wt%, up to 25 wt%, most preferably up to 10 wt% per total amount of the pharmaceutical composition.

The pharmaceutical composition of the present invention may also comprise a pharmaceutically acceptable binder to add cohesiveness to powders thereby providing the necessary bonding to form granules, which under compaction form a compact mass as tablet such as cellulose, microcrystalline cellulose, hydroxyethylcellulose, hydroxypropylcellulose, polyvinyl pyrrolidone, polyethylene glycol, maltodextrine, pregelatinized starch or a combination thereof. In one embodiment, the pharmaceutical composition comprises at least one binder in an amount of at least of up to 50 wt%, up to 25 wt%, up to 10 wt%, most preferably up to 5 wt% per total amount of the pharmaceutical composition.

The pharmaceutical composition of the present invention may also comprise a pharmaceutically acceptable disintegrant agent to accelerate disintegration of the solid dosage forms, such as sodium croscarmellose, sodium starch glycolate, crospovidone or a combination thereof. In one embodiment, the pharmaceutical composition comprises at least one disintegrant in an amount of at least of up to 20 wt%, up to 15 wt%, up to 10 wt%, most preferably up to 5 wt% per total amount of the pharmaceutical composition.

In some embodiments, the pharmaceutical composition further contains a pharmaceutically acceptable glidant to improve flow properties of the blend, such as colloidal silicon dioxide, talc, or a combination thereof. In one embodiment, the pharmaceutical composition comprises at least one glidant in an amount of up to 15 wt%, up to 10 wt%, up to 5 wt%, most preferably up to 1 wt% per total amount of the pharmaceutical composition.

The pharmaceutical composition of the present invention also contains a pharmaceutically acceptable lubricant to reduce ejection force during compression and avoid sticking. Such lubricants are selected among pharmaceutically acceptable fatty acid esters such as magnesium stearate, sodium stearyl fumarate, stearic acid, hydrogenated oil, or any combination thereof. In one embodiment, the pharmaceutical composition comprises at least one lubricant in an amount of amount of up to 10 wt%, up to 5 wt%, up to 3 wt%, up to 2 wt%, most preferably up to 1 wt% per total amount of the pharmaceutical composition.

The pharmaceutical composition of the present invention also contains a pharmaceutically acceptable film coating comprising film former, softener, wetting agent, opaque and/or colored pigments. Such film formers are selected among pharmaceutically acceptable polymers such as cellulose derivatives, vinyl polymers or polyacrylate/polymethacrylate polymers to provide a non-functional film coating or a film coating, which protects from moisture or the combination of both. In one embodiment, the pharmaceutical composition comprises at least one film-coating in the amount up to 10 wt%, up to 8 wt%, up to 6 wt%, up to 4 wt%, most preferably up to 2 wt% per total amount of the pharmaceutical composition. A conventional film-coating process may be used to produce the film coating, e.g. a method comprising spraying a solution or dispersion of the above ingredients in a suitable solvent (water or organic solvent) onto tablet cores or capsules in a perforated pan coater.

### Methods of Use

Also provided is the amorphous Vamorolone of the present invention for use in methods for treating a disease having a significant and pathologic inflammatory component that can be addressed by inhibition of NF-κB in a human or animal subject in need of such treatment comprising administering to the subject an amount of a suspension described herein effective to reduce or prevent the disease in the subject, optionally in combination with at least one additional agent suitable to treating the disease that is known in the standard of care.

Specific diseases to be treated by the compounds, compositions, and methods disclosed herein include acid-induced lung injury, acne (PAPA), acute respiratory distress syndrome, ageing, AIDS, HIV-1, alcoholic hepatitis, alcoholic liver disease, allergic bronchopulmonary aspergillosis, Alzheimer's disease, amyotrophic lateral sclerosis, angina pectoris, anhidrotic ectodermal dysplasia-ID, ankylosing spondylitis, antiphospholipid syndrome, aphthous stomatitis, appendicitis, arthritis, asthma, allergen induced asthma, non-allergen induced asthma, atherosclerosis, atopic dermatitis, autoimmune diseases, Behcet's disease, Bell's palsy, Blau syndrome, bronchiolitis, cancer, cardiac hypertrophy, catabolic disorders, cataracts, cerebral aneurysm, chronic heart failure, chronic lung disease (including of prematurity), chronic obstructive pulmonary disease, colitis, ulcerative colitis, complex regional pain syndrome, connective tissue diseases, Crohn's disease, cryopyrin-associated periodic syndromes, cyrptococcosis, cystic fibrosis, deficiency of the interleukin-1-receptor antagonist (DIRA), dermatitis, dermatomyositis, endometriosis, endotoxemia, familial amyloidotic polyneuropathy, familial cold urticaria, familial Mediterranean fever, fetal growth retardation, glaucoma, glomerular disease, glomerular nephritis, gut diseases, head injury, headache, hearing loss, heart disease, Henoch-Scholein purpura, hepatitis, hereditary periodic fever syndrome, herpes zoster and simplex, Huntington's disease, hyaline membrane disease, hypercholesterolemia, hyperimmunoglobulinemia D with recurrent fever (HIDS), hypoplastic and other anemias, incontinentia pigmenti, infectious mononucleosis, inflammatory bowel disease, inflammatory lung disease, inflammatory neuropathy, inflammatory pain, irritant-induced inflammation, plant irritant-induced inflammation, poison ivy/urushiol oil-induced inflammation, chemical irritant-induced inflammation, bee sting-induced inflammation, insect bite-induced inflammation, ischemia/reperfusion, kidney disease, kidney injury caused by parasitic infections, leptospiriosis, leukemia, lung injury, lupus, lupus nephritis, lymphoma, meningitis, mesothelioma, Muckle-Wells syndrome (urticaria deafness amyloidosis), multiple sclerosis, muscle wasting, muscular dystrophy, mycosis fungoides, myelodysplastic syndrome, myocarditis, myositis, nasal sinusitis, necrotizing enterocolitis, neonatal onset multisystem inflammatory disease (NOMID), nephrotic syndrome, neuritis, neuropathological diseases, obesity, ocular allergy, osteoarthritis, otitis media, Paget's disease, pain, pancreatitis, Parkinson's disease, pericarditis, periodic fever, periodontitis, peritoneal endometriosis, pertussis, pharyngitis and adenitis (PFAPA syndrome), pneumocystis infection, polyarteriitis nodosa, polycystic kidney disease, polymyositis, psoriasis, psychosocial stress diseases, pulmonary disease, pulmonary fibrosis, pulmonary hypertension, pyoderma gangrenosum, pyogenic sterile arthritis, renal disease, retinal disease, rheumatic disease, rheumatoid arthritis, sarcoidosis, seborrhea, sepsis, silica-induced diseases, Sjogren's syndrome, skin diseases, sleep apnea, solid tumors, spinal cord injury, stroke, subarachnoid hemorrhage, sunburn, burns, thrombocytopenia, TNF receptor associated periodic syndrome (TRAPS), toxoplasmosis transplant, organ transplant, tissue transplant, traumatic brain injury, tuberculosis, type 1 diabetes, type 2 diabetes, uveitis.

In some embodiments, the disease is chosen from acute lymphocytic leukemia, Addison's disease, adrenal hyperplasia, adrenocortical insufficiency, allergic conjunctivitis, alopecia, amyloidosis, angioedema, anterior segment inflammation, autoimmune hepatitis, Behcet's syndrome, berylliosis, bone pain, bursitis, carpal tunnel syndrome, chorioretinitis, chronic lymphocytic leukemia, corneal ulcer, diffuse intrinsic pontine glioma, epicondylitis, erythroblastopenia, gout, gouty arthritis, graft-versus-host disease, hemolytic anemia, Hodgkin's disease, hypercalcemia, hyperammonemia, hypoplastic anemia, idiopathic thrombocytopenic purpura, iritis, juvenile rheumatoid arthritis, keratitis, kidney transplant rejection prophylaxis, Loeffler's syndrome, mixed connective tissue disease, myasthenia gravis, mycosis fungoides, optic neuritis, pemphigus, pneumonia, pneumonitis, polychondritis, psoriasis, rheumatic carditis, severe pain, sickle cell, sickle cell anemia, Stevens-Johnson syndrome, temporal arteritis, tenosynovitis, thyroiditis, urticarial, Wegener's granulomatosis, and weight loss.

In some embodiments, the disease is asthma or chronic obstructive pulmonary disease.

In some embodiments, the disease is Sjogren's syndrome.

In some embodiments, the disease is arthritis.

In some embodiments, the disease is muscular wasting.

In some embodiments, the muscular wasting disease is muscular dystrophy.

In some embodiments, the muscular dystrophy is chosen from Duchenne muscular dystrophy, Becker muscular dystrophy, limb girdle muscular dystrophy, congenital muscular dystrophy, facioscapulohumeral muscular dystrophy, myotonic muscular dystrophy, oculopharyngeal muscular dystrophy, distal muscular dystrophy, and Emery-Dreifuss muscular dystrophy.

In some embodiments, the muscular dystrophy is Duchenne muscular dystrophy.

In certain embodiments, crystalline Vamorolone is administered at a dose of from 0.1 to 500 mg/kg per day, preferably 0.5 to 20 mg/kg per day, more preferably 1 to 10 mg/kg per day. Most preferably, crystalline Vamorolone is administered at a dose of from 2 to 6 mg/kg per day. In some embodiments, the dose range is 0.25 to 6.0 mg/kg/day, such as 0.25 mg/kg/day, 0.75 mg/kg/day, 2.0 mg/kg/day, or 6.0 mg/kg/day. The dose range for adult humans is generally from 5 mg to 2 g/day.

### EXAMPLES

### Example 1 - Synthesis of Vamorolone in US 2020/0281942

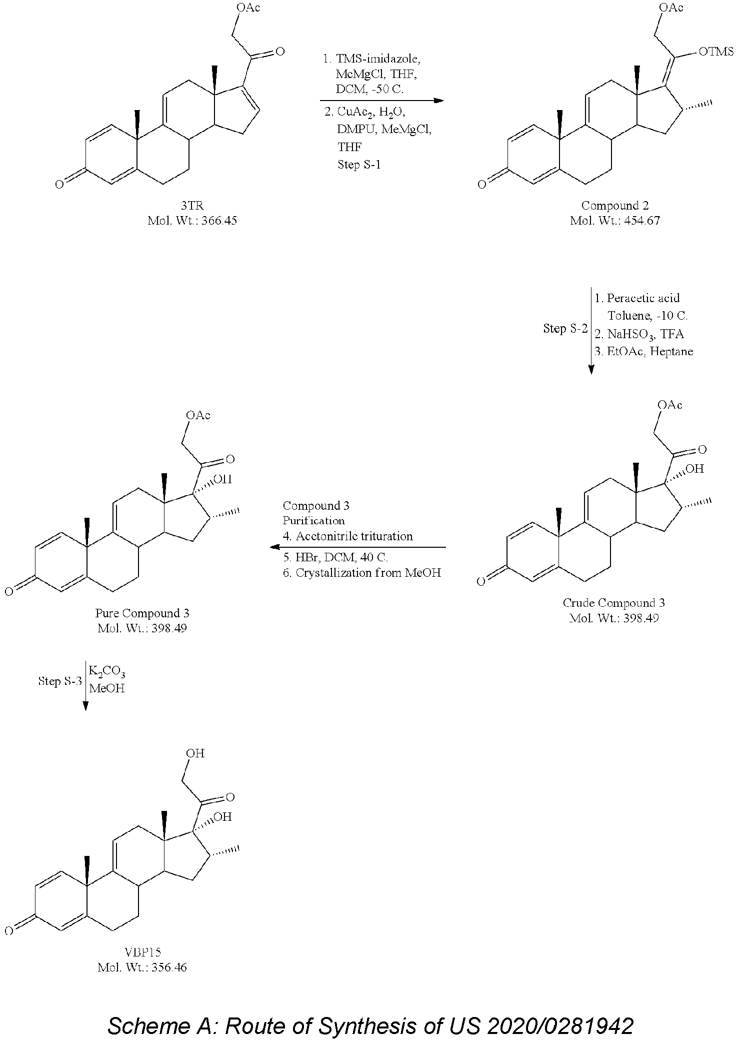

### 1.1 Step 1: Compound 2 Preparation

3-TR (100 g, 273 mmol), dichloromethane (DCM, 500 mL) and tetrahydrofuran (THF, 400 mL) were charged to a reaction flask under nitrogen. To this was charged trimethylsilyl imidazole (TMS-imidazole, 65.3 g, 466 mmol, 1.7 eq). The resulting mixture was stirred at room temperature for 3 hours.

In a separate flask, copper acetate monohydrate (5.4 g, 27 mmol), tetrahydrofuran (400 ml) and 1,3-dimethyl-3,4,5,6-tetrahydro-2(1H)-pyrimidinone (DMPU, 53.3 g, 416 mmol) were combined and stirred at room temperature for approximately 3 hours. The blue mixture was subsequently cooled to -50° C., and to this was added methyl magnesium chloride solution (27 ml, 3.0 M in THF, 82 mmol) dropwise. After 30 minutes, the mixture had formed a deep blue, sticky "ball."

The 3-TR/TMS-imidazole mixture was cooled to -50° C. and to this was charged the copper acetate/DMPU solution above via cannula. The residual sticky mass from the copper acetate/DMPU mixture was dissolved using DCM (50 mL) and also transferred.

Methyl magnesium chloride (123.2 mL, 3.0 M solution in THF, 368 mmol) was added dropwise over 45 minutes to the combined reaction mixtures, which were then allowed to stir for 2 hours at -50° C. Subsequent HPLC analysis showed complete consumption of starting material. The mixture was allowed to warm to room temperature overnight, with stirring.

Toluene (800 mL) was added to the mixture, followed by 5% acetic acid solution (600 mL). The aqueous layer was removed and discarded. The acetic acid wash was repeated. The organic layer was washed with brine (400 mL), 5% sodium bicarbonate solution (400 mL×2), followed by a brine wash (400 mL). The organic solution was dried over sodium sulfate, then concentrated to dryness under reduced pressure. The product was recovered as a viscous, light golden oil. Mass recovery was 146 grams (119% of theoretical).

### 1.2 Step 2 Compound 3 Preparation

Compound 2 (92 g, 202 mmol) and toluene (1000 mL, 10.9 vol) were charged to a reaction flask under nitrogen and the solution was cooled to -10° C. A 32 wt % solution of peracetic acid in acetic acid (60 mL, 283 mmol, 1.4 eq) was added dropwise over about 30 min maintaining the temperature at -10° C. The reaction was held for approximately 20 h (HPLC showed 75% Cmpd 3, Cmpd 2 1.5%, 6% diastereomer; 5% epoxide). Starting at -10° C., a 20% aqueous solution of sodium bisulfite (920 mL, 10 vol) was added carefully via addition funnel, keeping the temperature below 10° C. Trifluoroacetic acid (16 mL, 202 mmol, 1 eq) was added and the mixture was held for 3 h at 0-5° C. to complete desilylation (endpoint by HPLC). The lower aqueous layer was drained, and the organic layer was washed with a saturated solution of sodium bicarbonate (3×250 mL), followed by water (1×250 mL), and brine (1×150 mL). The organic layer was then dried over Na₂SO₄, filtered and concentrated to a pasty solid (89 g). The residue was taken up in 1.5 vol of EtOAc and transferred to neat heptane (19 vol) to precipitate crude Cmpd 3 as an off-white solid (50 g, 62.5% yield; HPLC 79% Cmpd 3, 5.6% epoxide, 1.7% diastereomer). The crude Cmpd 3 (48.5 g) was triturated in hot acetonitrile (2 vol) at 60° C. for 4 h, and then gradually cooled to ambient temperature overnight. The mixture was filtered using the recycled filtrate to rinse and wash the wet cake. After drying, the recovery was 64.3% (31.2 g; HPLC 93.5% Cmpd 3, 3.3% epoxide). To remove the epoxide impurity, the Cmpd 3 was dissolved in DCM (250 mL, 8 vol) and a solution of 48% HBr in water was added (7.5 mL). The mixture was heated at 40° C. for 1 h (HPLC<0.3% epoxide). The mixture was cooled and transferred to a separatory funnel. The lower aqueous layer (brown) was removed and the upper organic layer was washed with water (200 mL), saturated NaHCO₃ (150 mL), and brine (100 mL). The organic layer was dried over Na₂SO₄, filtered, and concentrated to a tan foam (32 g, ^{~}100% recovery). Methanol (64 mL, 2 vol) was added to the 32 g foam forming a slurry. To this was added a 1:1 solution of MeOH:water (60 mL, 2 vol) dropwise. The slurry cooled to slightly below ambient temperature and filtered using recycled filtrate to rinse and wash the wet cake. The solids were dried to constant weight, affording 26.1 g Cmpd 3 (81% recovery; HPLC 97.8%). The overall yield for Step 2 was 32.5%.

### 1.3 Step 3: VBP15 Preparation

Compound 3 (26 g, 65 mmol) and MeOH (156 mL, 6 vol) were mixed in a reaction flask and cooled to 0-5° C. A solution of K₂CO₃ (9.9 g, 72 mmol, 1.1 eq) in water (65 mL) was added dropwise, and the mixture was allowed to gradually warm to ambient temperature overnight. Analysis by HPLC showed 2.5% SM and another 5 mol % K₂CO₃ was added and the mixture stirred for another day (HPLC endpoint 1.1% Cmpd 3). The mixture was neutralized to pH 7 with 1.5 M HCl (53 mL) and ^{~}25% of the MeOH (30 g) was removed under vacuum to maximize recovery. After stirring for 2 days, the product was isolated by filtration using the recycled filtrate to aid transferring the wet cake to the funnel. The wet cake was dried under vacuum, affording 19.3 g VBP15 (83% yield) as an off-white powder. Analysis of the solids by HPLC showed 98.8% purity with 0.6% Cmpd 3 as the only major impurity.

### Example 2: Preparing an amorphous solid dispersion of Vamorolone

### 2.1 Amorphous Vamorolone stabilized by a polymer

The solubility of Vamorolone is limited to about 40 µg/ml in aqueous solution at 37°C.

In order to reduce the administered dose to the patient, a stable form of Vamorolone with increased solubility when compared to crystalline drug is needed.

Since various attempts in improving crystalline Vamorolone have failed, the present inventors investigated methods for obtaining stable amorphous forms of Vamorolone as amorphous solid dispersions to increase solubility.

However, this turned out to be difficult. Amorphous solids tend to crystallize into an energetically more stable crystalline form, which typically creates issues with solid oral dosage formulation stability, which is undesirable.

In preliminary attempts, amorphous Vamorolone could be obtained by cryo-milling, however, this material was found to recrystallize within days at room temperature in a closed container.

Spray drying and hot melt extrusions are common techniques employed for the production of amorphous solid dispersions. In such dispersions, the amorphous state is stabilized by a suitable polymer.

### 2.2 Screening for the best polymer in a first stage

In addition, since spray drying requires the drug and the polymer to be dissolved, screening for the best solvent system was also performed.

The solubility of micronized Vamorolone was assessed in several typical organic solvent systems suitable for use in spray drying.

A quantity of 10 mg of Vamorolone was dispensed into a 2 mL HPLC vial. The experimental design was to add the test solvent in aliquots, 200 µl and 100 µl. After each aliquot addition the sample was to be mixed on a vortex mixer for 1 minute and solubility assessed visually. Observations were also made after one hour of solvent addition.

Results of the solvent solubility screening for the drug substance Vamorolone are summarized in the table 1 below.

| **Solvent Systems used** | **200 µl** | **+100 µl** | **Observation after solubility assessment** | **Observation 1 hour after solubility assessment** |
|---|---|---|---|---|
| Methanol | × | ✔ | Clear | Clear |
| Ethanol | × | × | Hazy | Hazy |
| Isopropanol | × | × | Hazy | Hazy |
| Acetone | × | × | Hazy | Hazy |
| Ethyl Acetate | × | × | Hazy | Hazy |
| DCM | × | ✔ | Clear | Clear |
| Water | × | × | Hazy | Hazy |
| DCM:Methanol (1:1) | ✔ | | Clear | Clear |
| DCM:Methanol (1:3) | ✔ | | Clear | Clear |

### Table 1: solvent solubility screening

Both DCM (dichloromethane):methanol (1:1) and (1:3) solvent systems resulted in 50 mg/ml clear solution.

As a next step, the solubility of the polymers was assessed in the DCM:methanol (1:3) solvent system. A quantity of 10 mg of the polymer was dispensed into a 2 mL HPLC vial. The experimental design was to add the solvent system in aliquots of 100 µl. After each aliquot addition, the sample was mixed on a vortex mixer for 1 minute and solubility assessed visually.

The results of the assessment are summarized in the table below.

| **Polymer** | **100 µl** | **+100 µl** |
|---|---|---|
| HPMCAS-M | clear but very thick | Clear |
| HPMCAS-L | clear but very thick | Clear |
| HPMC-E3 | Clear | |
| Eudragit L100 | Clear | |
| Kollidon K30 | Clear | |
| Kollidon VA64 | Clear | |

### Table 2: solubility results

All the polymers resulted in 50mg/ml clear solution.

### 2.3 Spray dried prototypes

On the basis of the initial screening activities, spray dried prototypes (SDD) were obtained using conventional parameters.

Spray dying was performed under an inert atmosphere (N2) on a Büchi B-290 with a Büchi B-295 Inert Loop unit. Each run was performed under the same target spray drying parameters. DCM/methanol (1:3) was used as the solvent for all formulations. In all cases, a total of 15.0 g solid content was spray dried. The processing details are summarized in the table 3 below. Secondary drying was performed at 50°C under vacuum for 3-4 days.

**Table 3: processing details**

| **Parameter** | **HMPCAS-M 66.6% API** | **HMPCAS-M 50% API** | **HMPCAS-M 33% API** | **HMPC-E3 50% API** | **HMPC-E3 33.3% API** | **PVP VA64 33.3% API** |
|---|---|---|---|---|---|---|
| Feedstock solids loading (% w/w) | 7.5 | 9.8 | 13.9 | 9.8 | 13.9 | 13.9 |
| Aspirator (%) | 100 | | | | | |
| Inert Loop (°C) | -20 °C | | | | | |
| Inlet Temp. (°C) | 60 | 50 | 60 | 50-60* | 60 | 60 |
| Outlet Temp. (°C) | 42 | 34 | 40 | 35-41* | 42 | 43 |
| Flow meter (mm) | 35 | 35 | 35 | 35 | 35 | 35 |
| Atomization (bar) | 0.3 | 0.3 | 0.3 | 0.3 | 0.3 | 0.3 |
| Feed rate (g/min) | 6.3 | 6.1 | 6.2 | 6.5 | 6.2 | 6.4 |
| Yield (%) | 81 | 79 | 53 | 72 | 68 | 73 |

| | | | | | | |
|---|---|---|---|---|---|---|
| *the inlet temperature was increased from 50 to 60°C to increase the outlet temperature to above 40 °C. | | | | | | |

The amorphous state of Vamorolone in the SDD prototypes was confirmed by XRPD and scanning electron microscopy, and DSC. Particle size distribution of Vamorolone amorphous dispersion was determined by laser diffraction particle size analysis with d10 1.4µm, d50 7.5µm and d90 17.5µm. Specific surface as measured by BET surface area analysis was 1.088m²/g.

### 2.4 Kinetic solubility data for spray dried prototypes

SDD prototypes were evaluated for kinetic solubility in FaSSIF (Fasted State Simulated Intestinal Fluid, pH = 6.5) using the Pion µDissolution monitor with a tip path length of 2 mm. Data were collected using AUPro software over a period of 18 hours in 20 mL of each media, heated to 37 ± 2 °C. A stir speed of 250 rpm for the duration of the experiment.

For HPMAS-M, the amorphous solid dispersion of Vamorolone exhibited a surprisingly enhanced kinetic solubility of more than about 4-fold when compared to the crystalline drug, as shown in the table 4 below.

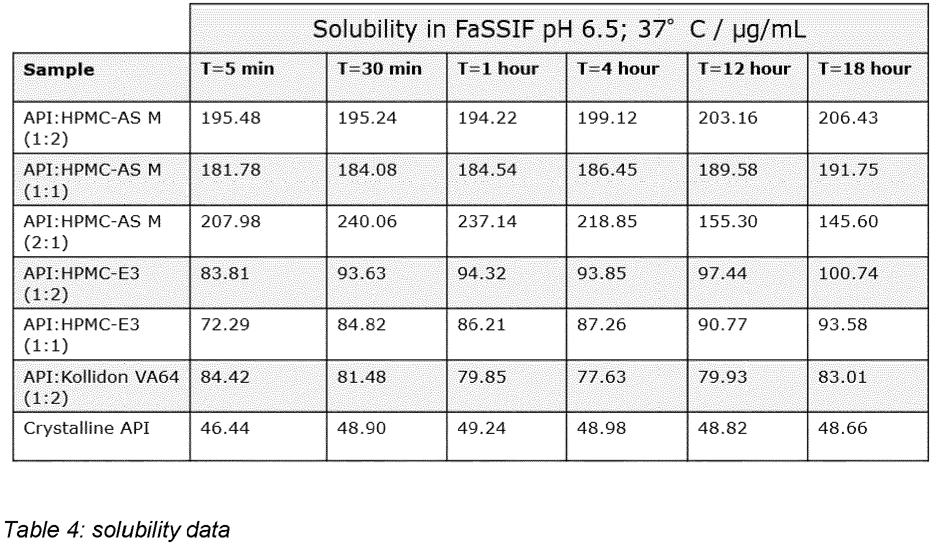

- All SDD formulations allowed for a higher concentration of Vamorolone in FaSSIF to be reached when compared to the crystalline Vamorolone.
- The performance of each polymer could be ranked as such: HPMC-AS M > HPMC-E3 > Kollidon VA64
- The ability of Vamorolone to remain solubilized is affected by the different drug loadings. For HPMC-AS M at higher polymer loadings (and hence lower Vamorolone loadings), the dissolution profile over time remains flat and no precipitation of Vamorolone is seen. For the SDDs which feature higher Vamorolone loadings and lower polymer loadings, Vamorolone is at a much higher risk of precipitation and the stabilization effect of the polymer to hold Vamorolone in solution decreases with time.

Figure 2 shows on overlay of kinetic solubility profiles obtained for eight spray dried amorphous solid dispersion prototypes of Vamorolone ("API"). In fasted state simulated intestinal fluid (FaSSIF). The solubility profile of micronized crystalline Vamorolone has also been included for comparison purposes. All prototypes exhibited an increased solubility compared to crystalline Vamorolone, however, the performance of the different polymers was unexpectedly diverse, being ranked as: HPMC-AS M > HPMC-E3 > Kollidon VA64. In addition, the ability of Vamorolone to remain solubilized by the polymer in a supersaturated solution is affected by the different drug loadings. For the polymer with the best performance, HPMC-AS M, at higher polymer loadings (and hence lower API loadings), the solubilized state was maintained and no precipitation of Vamorolone was observed. At higher drug loads of more than around 67% or more, Vamorolone is at a much higher risk of precipitation as the drug to polymer ratio is reduced and less polymer is available to stabilize the drug. Accordingly, solubility decreases with time.

Figure 3 shows comparative kinetic solubility profiles obtained for crystalline Vamorolone, spray dried amorphous solid dispersion prototype (Vamorolone: HPMCAS M 1:1) and a physical mixture of Vamorolone and HPMCAS M 1:1. The presence of the polymer alone does not result in an increase of solubility as seen for the solid dispersion.

### 2.5 HME (hot melt extrusion)

The feasibility of preparing amorphous solid dispersions of Vamorolone was further evaluated by using hot melt extrusion. In preliminary trials, miscibility was assessed by DSC (differential scanning calorimetry).

The following polymer systems were chosen because of the wide processing temperature range (a wider difference between Tg and the degradation temperature):
- HPMCAS-M (Tg 120°C)
- HPMCAS-L (Tg 120°C)
- Kollidon VA64 (Tg 101°C)
- Kollidon VA64/HPMC-E3 (75/25)
- Kollidon VA64/poloxamer 188 (97.5/2.5)

For HPMCAS-M and HPMCAS-L, even 20% Vamorolone blends still showed crystallinity post DSC run. Similar result was observed for 40% Vamorolone in HPMC-E3 blend. These polymers were thus found to be unsuitable for further HME experiments.

The best results were obtained for Kollidon VA64, with drug loads of 50% as well as 60% Vamorolone blends were found to be completely amorphous by XRPD post DSC runs, indicating miscibility of the Vamorolone at least up to 60% Vamorolone load.

### 2.6. HME prototype Preparation

The scale of manufacture was 30g of blend. The blend for extrusion was manufactured by weighing the required amounts of each material and placing them in the blending container. The blending was performed on a Pharmatech MB005 blender. Hot Melt Extrusion (HME) was performed on a Thermo Scientific Process 11 extruder fitted with a 2 mm die and carrying out trials using two standard screw configurations and processing conditions.

The experiments resulted in opaque/crystalline extrudates, in which the drug substance experienced significant degradation. The Vamorolone in VA64 blends showed remarkable crystallization rendering HME an undesirable approach to stabilize Vamorolone in the amorphous form.

Figure 1a shows the crystallization pattern of Vamorolone in VA64 blends. As a comparison Figure 1b is presented showing amorphous Vamorolone stabilized in formulations obtained by spray drying.

It was considered that for the Vamorolone/VA64 system, HME is not a feasible technology to prepare stable amorphous solid dispersions. Moreover, other polymers were not attractive enough to be further explored due to the relatively low achievable drug load.

Overall, HME trials unexpectedly showed either a significant degradation of the resulting extrudates or lack of miscibility of the Vamorolone in the polymers, suggesting this not as a feasible technology to produce amorphous solid dispersions of Vamorolone.

### 2.7. Stability Studies of the spray drying obtained (SDD) Prototypes

Based upon the characterization and kinetic solubility data, the following batches of prototype SDDs were set down for a 4-week stability study to determine the physical and chemical stability of the amorphous solid dispersions of Vamorolone.

**Table 5: SDD Description**

| SDD Description | % Vamorolone Loading | Batch Number |
|---|---|---|
| 2:1 Vamorolone : HPMC-AS M | 66.6 | 7170/81 |
| 1:1 Vamorolone : HPMC-AS M | 50.0 | 7170/57 |
| 1:2 Vamorolone : HPMC-AS M | 33.3 | 7244/01 |
| 1:1 Vamorolone : HPMC-E3 | 50.0 | 7170/65 |

All samples were set down at 25°C / 60% relative humidity (Closed) and 40°C / 75% relative humidity (Open) stability conditions for the duration of the study. In addition, 40°C / 75% relative humidity (Closed) samples were also set down for the 55% and 60% SDDs to assess the impact of humidity, data not shown. SDDs were stored in Type I glass borosilicate vials, which were stored upright for the duration of the study.

Figure 4 shows XRPD data for HPMC-E3 (1:1), HPMCAS-M(1:2), HPMCAS-M(1:1) and HPMCAS-M(2:1) after 4 weeks at 25°C and 60% relative humidity (closed), and 40°C and 75% relative humidity (open).

Out of all the SDDs, the 1:1 API/HPMC-E3 SDD showed the worst results in terms of physical stability, with clear signs of crystallization at 40°C/75% relative humidity, followed by the sample corresponding to HPMCAS-M (2:1).

No crystallization was observed for HPMCAS-M(1:2) and HPMCAS-M(1:1) under all conditions tested.

Chemical stability was satisfactory in all cases, with total impurities content below 1.0% in all batches for all conditions after 4 weeks.

### 2.8. Conclusions

HME trials showed either a significant degradation of the resulting extrudates or lack of miscibility of the Vamorolone in the polymers, suggesting this not as a feasible technology to produce amorphous solid dispersions.

Spray drying trials showed promising results for SDD prototypes under specific conditions. The amorphous solid dispersion containing Vamorolone: HPMCAS-M (1:1) was considered to be the best balance in terms of stability and drug load for further development.

### Example 3: Solid dosage forms.

The amorphous solid dispersions described above were obtained in the form of fine powders that can be characterized by PSD, density and other techniques known to those skilled in the art.

PSD for the HPMCAS-M(1:1) batch was measured by laser diffraction (dry method, Malvern Mastersizer 3000), with following exemplary results: D10: 1.419 µm, D50: 7.528 µm and D90: 17.509 µm.

True density was determined using the AccuPyc II 1340 pycnometer: 1.27 g/ml. In addition, Compaction simulation studies were performed by conducting a Heckel test. The aim of the Heckel test is to compress the material under controlled conditions to derive the yield pressure of the bulk material. A known weight of material is compressed within a 10 mm diameter die with flat faced punches moving at a set speed. The force on the punch is accurately measured at frequent intervals whilst the displacement of the punches is used to calculate the volume of the powder. The yield pressure is calculated at slow and fast punch speeds to assess the time dependent component to deformation of the material. Based on this study, the ASD powder (obtained by spray drying, i.e. SDD) was classified as soft ductile.

Moreover, if formulated into a tablet, the strength results suggested that the Vamorolone ASD will be able to contribute to the overall strength of the tablet and therefore high concentrations are theoretically possible.

The ASD manufactured as described before can be used for the manufacture of solid dosage forms such as tablets, coated tablets, mini-tablets, pellets, granules or capsules. Said dosage forms can be obtained by employing standard manufacturing processes and equipment. Typical unit operations that might be applied include, but are not limited to, blending, sieving, milling, roller compaction, slugging, tableting, extrusion and spheronization, capsule filling and film coating, among others.

Standard pharmaceutically acceptable excipients are processed together with the ASD to obtain such dosage forms.

The table 6 below displays a tablet formulation containing ASD of Vamorolone : HPMCAS-M (1:1). Tablets were manufactured by dry granulation and the obtained granules were compressed into different tablet weights to achieve different dosage strengths.

**Table 6: tablet formulation**

| **Dosage strength of Vamorolone** | **Amount per tablet (mg)** | | |
|---|---|---|---|
| | **75 mg** | **150 mg** | **300 mg** |
| ASD | 150 | 300 | 600 |
| Microcrystalline cellulose PH 101 | 50 | 100 | 200 |
| Lactose monohydrate | 35 | 70 | 140 |
| Croscarmellose sodium | 10 | 20 | 40 |
| Magnesium stearate | 1.25 | 2.5 | 5 |
| **Tablet weight** | 250 | 500 | 1000 |

## Claims

1. An amorphous solid dispersion comprising Vamorolone and at least one pharmaceutically acceptable polymer, wherein the pharmaceutically acceptable polymer is selected from hydroxypropylmethylcellulose (HPMC), hydroxypropylcellulose (HPC), povidone, copovidone (polyvinylpyrrolidone-vinyl acetate copolymer) and hydroxypropylmethylcellulose acetate succinate (HPMCAS) of grade L, M or H.

2. The amorphous solid dispersion of claim 1, wherein the Vamorolone is present in an amount of at least 20 wt% by weight of the amorphous solid dispersion.

3. The amorphous solid dispersion of any of claim 1-2, comprising Vamorolone in an amount between 40 and 60 wt% by weight of the amorphous solid dispersion and total polymer in an amount between 40 and 60 wt% by weight of the amorphous solid dispersion.

4. The amorphous solid dispersion of any of claim 1-3, wherein the composition comprises a polymer selected from selected from HPMC, HPMCAS-L, HPMCAS-M and HPMCAS-H.

5. The amorphous solid dispersion of any of claims 1-4 comprising other ingredients in an amount of not more than 3 wt% by weight of the amorphous solid dispersion.

6. The amorphous solid dispersion of any of claims 1-5, wherein the amorphous solid dispersion of Vamorolone is obtained by spray drying.

7. A pharmaceutical composition comprising the amorphous solid dispersion of any of claims 1-6 and a pharmaceutically acceptable excipient.

8. The pharmaceutical composition of claim 7, wherein the pharmaceutical composition comprises Vamorolone in an amount of at least 20 wt% by weight of the pharmaceutical composition.

9. The pharmaceutical composition of claim 7 or 8, further comprising a filler, a binder, a disintegrant, a lubricant, a glidant and/or a film coating.

10. The pharmaceutical composition of any of claims 6-9, which is a tablet or capsule.

11. Use of a polymer for improving the solubility of Vamorolone in an aqueous medium by dispersing Vamorolone in amorphous form in the polymer, wherein the polymer is selected from hydroxypropylmethylcellulose (HPMC), hydroxypropylcellulose (HPC), povidone, copovidone (polyvinylpyrrolidone-vinyl acetate copolymer) and hydroxypropylmethylcellulose acetate succinate (HPMCAS) of grade L, M or H.

12. A method of preparing the amorphous solid dispersion of any of claims 1-6 by spray drying.

## Patentansprüche

1. Eine amorphe feste Dispersion, umfassend Vamorolon und mindestens ein pharmazeutisch annehmbares Polymer, wobei das pharmazeutisch annehmbare Polymer ausgewählt ist aus Hydroxypropylmethylcellulose (HPMC), Hydroxypropylcellulose (HPC), Povidon, Copovidon (Polyvinylpyrrolidon-Vinylacetat-Copolymer) und Hydroxypropylmethylcelluloseacetatsuccinat (HPMCAS) der Qualität L, M oder H.

2. Die amorphe feste Dispersion nach Anspruch 1, wobei das Vamorolon in einer Menge von mindestens 20 Gew.-% der amorphen festen Dispersion vorhanden ist.

3. Die amorphe feste Dispersion nach einem der Ansprüche 1 bis 2, umfassend Vamorolon in einer Menge zwischen 40 und 60 Gew.-% der amorphen festen Dispersion und Gesamtpolymer in einer Menge zwischen 40 und 60 Gew.-% der amorphen festen Dispersion.

4. Die amorphe feste Dispersion nach einem der Ansprüche 1 bis 3, wobei die Zusammensetzung ein Polymer umfasst, das aus HPMC, HPMCAS-L, HPMCAS-M und HPMCAS-H ausgewählt ist.

5. Die amorphe feste Dispersion nach einem der Ansprüche 1-4, die andere Bestandteile in einer Menge von nicht mehr als 3 Gew.-% der amorphen festen Dispersion enthält.

6. Die amorphe feste Dispersion nach einem der Ansprüche 1 bis 5, wobei die amorphe feste Dispersion von Vamorolon durch Sprühtrocknung erhalten wird.

7. Eine pharmazeutische Zusammensetzung, umfassend die amorphe feste Dispersion nach einem der Ansprüche 1 bis 6 und einen pharmazeutisch annehmbaren Hilfsstoff.

8. Die pharmazeutische Zusammensetzung nach Anspruch 7, wobei die pharmazeutische Zusammensetzung Vamorolon in einer Menge von mindestens 20 Gew.-% der pharmazeutischen Zusammensetzung umfasst.

9. Die pharmazeutische Zusammensetzung nach Anspruch 7 oder 8, die außerdem einen Füllstoff, ein Bindemittel, ein Sprengmittel, ein Gleitmittel und/oder eine Filmbeschichtung enthält.

10. Die pharmazeutische Zusammensetzung nach einem der Ansprüche 6-9, die eine Tablette oder Kapsel ist.

11. Die Verwendung eines Polymers zur Verbesserung der Löslichkeit von Vamorolon in einem wässrigen Medium durch Dispergieren von Vamorolon in amorpher Form in dem Polymer, wobei das Polymer ausgewählt ist aus Hydroxypropylmethylcellulose (HPMC), Hydroxypropylcellulose (HPC), Povidon, Copovidon (Polyvinylpyrrolidon-Vinylacetat-Copolymer) und Hydroxypropylmethylcelluloseacetatsuccinat (HPMCAS) der Qualität L, M oder H ausgewählt ist.

12. Ein Verfahren zur Herstellung der amorphen festen Dispersion nach einem der Ansprüche 1-6 durch Sprühtrocknung.

## Revendications

1. Dispersion solide amorphe comprenant du Vamorolone et au moins un polymère pharmaceutiquement acceptable, dans laquelle le polymère pharmaceutiquement acceptable est choisi parmi de l'hydroxypropylméthylcellulose (HPMC), de l'hydroxypropylcellulose (HPC), de la povidone, de la copovidone (copolymère polyvinylpyrrolidone-acétate de vinyle) et de l'acétate succinate d'hydroxypropylméthylcellulose (HPMCAS) de grade L, M ou H.

2. Dispersion solide amorphe selon la revendication 1, dans laquelle le Vamorolone est présent selon une quantité d'au moins 20 % en poids de la dispersion solide amorphe.

3. Dispersion solide amorphe selon l'une quelconque des revendications 1 et 2, comprenant du Vamorolone selon une quantité comprise entre 40 et 60 % en poids de la dispersion solide amorphe et un polymère total selon une quantité comprise entre 40 et 60 % en poids de la dispersion solide amorphe.

4. Dispersion solide amorphe selon l'une quelconque des revendications 1 à 3, dans laquelle la composition comprend un polymère choisi parmi HPMC, HPMCAS-L, HPMCAS-M et HPMCAS-H.

5. Dispersion solide amorphe selon l'une quelconque des revendications 1 à 4 comprenant d'autres ingrédients selon une quantité ne pas plus de 3 % en poids de la dispersion solide amorphe.

6. Dispersion solide amorphe selon l'une quelconque des revendications 1 à 5, dans laquelle la dispersion solide amorphe de Vamorolone est obtenue par séchage par pulvérisation.

7. Composition pharmaceutique comprenant la dispersion solide amorphe selon l'une quelconque des revendications 1 à 6 et un excipient pharmaceutiquement acceptable.

8. Composition pharmaceutique selon la revendication 7, dans laquelle la composition pharmaceutique comprend du Vamorolone selon une quantité d'au moins 20 % en poids de la composition pharmaceutique.

9. Composition pharmaceutique selon la revendication 7 ou 8, comprenant en outre une charge, un liant, un désintégrant, un lubrifiant, un agent de glissement et/ou un enrobage pelliculaire.

10. Composition pharmaceutique selon l'une quelconque des revendications 6 à 9, qui est un comprimé ou une gélule.

11. Utilisation d'un polymère pour améliorer la solubilité de Vamorolone dans un milieu aqueux en dispersant du Vamorolone sous forme amorphe dans le polymère, dans laquelle le polymère est choisi parmi de l'hydroxypropylméthylcellulose (HPMC), de l'hydroxypropylcellulose (HPC), de la povidone, de la copovidone (copolymère polyvinylpyrrolidone-acétate de vinyle) et de l'acétate succinate d'hydroxypropylméthylcellulose (HPMCAS) de grade L, M ou H.

12. Procédé de préparation de la dispersion solide amorphe selon l'une quelconque des revendications 1 à 6 par séchage par pulvérisation.
